# EUROPEAN PATENT SPECIFICATION

(11) **EP 2 529 388 B1**
(45) Date of publication and mention of the grant of the patent: **23.10.2019**
(21) Application number: 11705663.0
(22) Date of filing: 31.01.2011
(51) Int. Cl.: H01J 49/00

(54) **FRAGMENTATION REAGENTS FOR MASS SPECTROMETRY**
FRAGMENTIERUNGSREAGENZIEN FÜR MASSENSPEKTROMETRIE
RÉACTIFS DE FRAGMENTATION POUR UNE SPECTROMÉTRIE DE MASSE

(30) Priority: 04.08.2010 US 370577 P; 02.08.2010 GB 201013022; 26.03.2010 US 317739 P; 29.01.2010 GB 201001519
(43) Date of publication of application: 05.12.2012
(73) Proprietor: Micromass UK Limited, Wilmslow SK9 4AX (GB)
(72) Inventor: BROWN, Jeffery Mark, Hyde Cheshire SK14 6LT (GB); CAMPUZANO, Iain, Swinton Manchester M27 0BE (GB); BROUSMICHE, Darryl W., Marlborough, Massachusetts 01752 (US); DORSCHEL, Craig, Worcester, Massachusetts 01606 (US); WILLIAMS, Jonathan Paul, Neath South Wales SA11 3PH (GB); PRINGLE, Steven Derek, Darwen Lancashire BB3 3PS (GB); RICHARDSON, Keith, High Peak Derbyshire SK22 4PP (GB)
(74) Representative: Harding, Andrew Philip
(86) International application number: PCT/GB2011/050155
(87) International publication number: WO 2011/092515

(56) References cited:
- WO-A2-2006/023398
- WO-A2-2009/066087
- US-A1- 2005 199 804
- HARSHA P. GUNAWARDENA ET AL: "Electron Transfer versus Proton Transfer in Gas-Phase Ion/Ion Reactions of Polyprotonated Peptides", JOURNAL OF THE AMERICAN CHEMICAL SOCIETY, vol. 127, no. 36, 1 September 2005 (2005-09-01), pages 12627-12639, XP55000146, ISSN: 0002-7863, DOI: 10.1021/ja0526057 cited in the application
- RICHARD H. WILEY ET AL: JOURNAL OF THE AMERICAN CHEMICAL SOCIETY, vol. 70, no. 4, 19 April 1948 (1948-04-19) , pages 1560-1561, XP55000145, ISSN: 0002-7863, DOI: 10.1021/ja01184a080

## Description

### TECHNICAL FIELD

This invention relates to fragmentation reagents for mass spectrometry, particularly but not exclusively to reagents for electron transfer dissociation (ETD) reactions. The invention also relates to a method of analysis using the reagents and to apparatus for carrying out the method.

### BACKGROUND

In mass spectrometry, biomolecule ions are often fragmented prior to mass analysis. A particular method of biomolecule fragmentation entails reaction of biomolecule cations and reagent anions in quadrupole ion traps, in particular, in linear ion traps (LIT) based on a quadruple set of rod electrodes, which utilize radio-frequency (RF) fields for ion confinement. An ion-ion fragmentation reaction, in which electron transfer is assumed to be central to the reaction process is referred to as Electron-Transfer Dissociation (ETD).

Liang, Hager and McLuckey, Analytical Chemistry, Vol. 79, pages 3363-3370 (2007), described four methods of providing peptide ion ETD reactions in a LIT. Three of these methods entail allowing cations and/or anions to pass through the LIT so that only cations, anions or neither are confined in the LIT. These three methods use a DC potential barrier for axial confinement of ions of a single polarity. The reference to "axial" is a conventional reference to the lengthwise direction of a LIT The fourth method simultaneously confines ions of opposite polarities along the axial direction, through use of RF pseudopotential barriers or by application of unbalanced RF fields to the quadrupole rod set. An axial pseudopotential barrier is formed by application of RF oscillating potentials to containment lenses at the axial ends of the LIT.

Optimal ion flow control is difficult to achieve with single-polarity confinement because the DC potential barrier used for axial confinement of ions of a single polarity acts as an accelerating potential for the opposite-polarity ions flowing through the LIT. The kinetic energy difference between the flowing ions and the confined ions may be too high for some ion-ion reactions to favorably occur. The ion-ion reaction rate may beinversely proportional to the third power of the relative velocity between the ions. Thus, mutual storage of biomolecule cations and reagent anions may provide the lowest relative ion velocities, suitable for more efficient dissociation.

Mutual confinement typically requires more complex use of RF voltages. The barrier height of an RF-generated pseudopotential barrier is a function of the mass-to-charge (m/z) ratio of the ions being stored, thus restricting the mass range of analyte or reagent ions that can be mutually confined for ion-ion reactions.

As an alternative to using RF fields for mutual confinement, two separate quadrupole rod sets (i.e. two adjacent LITs) may be used to separately store cations and anions prior to allowing ions from one LIT to flow through the other LIT. Such an approach to mutual confinement increases the complexity and cost of a fragmentation device.

Successful ETD and other fragmentation methods also require suitable reagent compounds. Some known reagents such as azobenzene are relatively toxic materials and may be undesirable for other reasons.

According to a first aspect of the present invention, there is provided a use of a compound as a mass spectrometry electron transfer dissociation reagent according to claim 1.

In a preferred embodiment, the electron affinity has a positive value between 0.1 to 150kJ/mol, preferably 0.1 and 100 kJ/mol.

The reagent is preferably an aromatic compound wherein the aromatic nucleus is substituted with one or more substituents selected from the group consisting of cyano, nitroso, carboxyl, iodo, aldehyde and acetoxy.

The nucleus is preferably selected from the group consisting of: phenyl, pyridinyl, pyrimidinyl, triazolyl, styrenyl and picolinyl.

In alternative embodiments of the invention, the reagent may be an unsaturated aliphate compound, for example selected from the group consisting of ethylene, acetylene, acrylate wherein two or more unsaturated bonds are conjugated. Alternatively, a single unsaturated bond may be conjugated with a double bond of a substituent.

In a preferred embodiment of the invention, the reagent is selected from the group consisting of:-
1,4-dicyanobenzene
1,3-dicyanobenzene
1-4-para-nitrotoluene
1,3-benzenedicarboxaldehyde (isopthalaldehyde)
1,2 ortho-nitrosotoluene
benzaldehyde oxime
nitrosobenzene
3-pyridinecarbonitrile
ethyl cis (beta cyano) acrylate
1,2,4,5-cyanobenzene
1,4-benzenedicarboxaldehyde (terephthalaldehyde)
3 acetobenzonitrile
3-nitrobenzaldehyde
4-formyl benzonitrile

According to a second embodiment of the invention, the reagent may be selected from the group consisting of:-
1,2-dicyanoethylene
5-cyano-1,2,4-triazole (124-triazole-5-carbonitrile)
2,5-dicyanopyrimidine
3,5-dicyanopyridine
5-methylpyridine 3-carbonitrile
5-pyrimidinecarbonitrile
2,4-dicyanopyrimidine
3,5-dicyanotoluene
4-cyanostyrene (4-ethynylbenzonitrile)
benzaldehyde
2-cyanostyrene
1,3,4,5-cyanobenzene
3-methyl picolinonitrile
4-vinylpyridine
4-pyrimidine carbonitrile
2-pyrimidine carbonitrile
4-acetobenzonitrile
2-pyrazinecarbonitrile
3-acetopyridine
4-cyanonitrobenzene
3-chlorobenzaldehyde
3-cyanonitrobenzene
1,4-diacetylbenzene
1,2-dicyanobenzene
3-nitrosopyridine
4-acetopyridine
4-chloroacetophenone
3-chloroacetophenone
3,5-dichlorobenzonitrile
1,3-meta-nitrosotoluene
1,2,3-tricyanobenzene
3,5-chloroacetophenone
4-nitrosopyridine
3-amino-4-cyanopyridine
2-amino-4-cyano pyridine
2-nitrosopyridine
4-amino-2-cyanopyridine
1,3-cyclohexanedione
4-amino-5-pyrimidine carbonitrile
4,5-dicyanoimidazole
3-thiophene carbonitrile
2-cyanoethyl acrylate
diaminomaleonitrile
2-cyanothioacetamide
phthalic acid
4-chloropyridine
2,4,6-CHT-36-carbonitrile
acetyl malonitrile
ethyl-2-cyanoacrylate
2-cyano-3-methyl but-2-enoic acid
1,2-dicyanoethyne (acetylene dinitrile)

According to a second aspect of the invention there is provided a method of characterization of an analyte comprising the steps of providing a first supply of analyte cations using electrospray ionisation;providing a second supply of reagent anions using an ionisation source;combining the first and second supplies; and carrying out a mass spectrometry (MS) technique on the combined supplies;wherein the reagent is a reagent as disclosed in the first aspect of this invention.

Preferably the analyte is a biomolecule.

The invention finds particular application wherein the analyte is selected from a polypeptide, a protein, a post-translationally modified peptide or protein or mixtures thereof

The invention arises from the discovery that preferred monocyclic substituted aryl compounds provide relatively efficient fragmentation by ETD. For example, nitrosobenzene is a suitable ETD reagent. Use of such relatively low mass reagents in a stacked-electrode-based fragmentation device can avoid loss due to the low-mass cut-off that occurs in other ion storage devices (e.g. 3D ion traps),

The negative ion transfer dissociation may be used. This is termed nETD. In this mode, multiply deprotonated analyte anions react with reagent cations and the resulting product ions include a- and x- type fragments. The electron is transfered from the analyte to the reagent. Typical reagents for nETD are those that also work for ETD in that they are radical.

The reagents of this invention may be suitably employed with a wide variety of mass-spectrometry equipment and a wide variety of fragmentation devices. For example, rod-based linear ion traps may be used. Some embodiments provide mutual confinement of oppositepolarity ions in a linear fragmentation device that does not rely on axial pseudopotential barriers, unbalanced RF fields, or two adjacent ion traps. In a preferred embodiment, the fragmentation device includes a stacked ring ion guide. Such a device may comprise a linearly arranged series of electrodes, each electrode defining an aperture through which ions may pass. Preferably, ETD is implemented in stacked-electrode devices through use of travelling DC potential waves to control the axial location and/or movement of ions through the device.

Some stacked-ring embodiments include means for mutual confinement of opposite-polarity ions, for biomolecule fragmentation, using DC potentials for axial confinement of both polarity types. One such device includes a set of stacked-rings, such as those found in a stacked-ring ion guide (SRIG). The device may have DC voltage control means that provides simultaneous storage, mixing, and/or release of positive ions and negative ions, or electrons, through use of discrete potential wells. In an alternative embodiment, a stacked-electrode-based fragmentation device can also be used for mass-related analysis of the ions and/or fragments, and/or can selectively eject ions and/or fragments for further analysis by down-stream modules. With application of an axial magnetic field, mutual confinement of biomolecule ions and electrons supports biomolecule fragmentation via reaction of biomolecule ions and free electrons.

Preferred embodiments provide a means for mutually confining cations and anions to perform ETD without the disadvantages described above.

Preferred embodiments of the present invention relate to apparatus for mass spectrometry which may spatially manipulate and confine mixtures of ions with opposite-polarity charged particles, such as reagent ions and/or electrons, to permit fragmentation. Some embodiments of the invention are suitable for ETD ion-ion reactions.

Preferred stacked-ring based embodiments utilize one or more axially traveling DC potential fields to control the ETD process by moving ions into and/or out of the device to control the duration of an ETD reaction. Travelling waves may be used to move ions in either or both axial directions.

According to a preferred aspect of this invention there is provided a method of peptide analysis, comprising the steps of: providing a fragmentation device including stacked electrodes that each define an aperture, the apertures defining an ion-manipulation region and an axial direction of the fragmentation device;
providing positively charged analyte ions; providing negatively charged reagent ions formed from a reagent as disclosed in the first aspect of this invention;
allowing the analyte ions and reagent ions to interact and cause fragmentation of the analyte ions; and
causing at least a portion of the fragmented analyte ions to exit the ion-manipulation region.

The electron transfer dissociation (ETD) device for use in the method of this invention may include: a plurality of electrodes defining a reaction space; and a first device arranged and adapted to apply one or more first transient DC voltages or potentials or one or more first transient DC voltage or potential waveforms to at least some of the plurality of electrodes in order to drive or urge at least some first ions along and/or through at least a portion of the axial length of the ion guide in a first direction.

The plurality of electrodes may comprise a set or rods, such as a quadrupole, or a set of stacked electrodes for use with travelling waves.

The electron transfer dissociation device, for use in the method of this invention may be adapted so that: (a) in a mode of operation one or more first transient DC voltages or potentials or one or more first transient DC voltage or potential waveforms are applied to at least some of a plurality of electrodes in order to drive or urge at least some product or fragment ions along at least a portion of the axial length of the electrodes in a direction different to a first direction; and/or (b) in a mode of operation the one or more second transient DC voltage or potentials or one or more second transient DC voltage or potential waveforms are applied to at least some of the plurality of electrodes in order to drive product or fragment ions along and/or through at least a portion of the axial length of the electrodes in a direction different to the second direction.

According to a further aspect of this invention there is provided a method of performing an electron transfer dissociation reaction including the steps of: providing an electron transfer dissociation device including a plurality of stacked electrodes;
applying one or more first transient DC voltages or potentials or one or more first transient DC voltage or potential waveforms to at least some of the plurality of electrodes in order to drive at least some first ions along at least a portion of the axial length of the ion guide in a first direction; and applying one or more second transient DC voltages or potentials or one or more second transient DC voltage or potential waveforms to at least some of the plurality of electrodes in order to drive at least some second ions along at least a portion of the axial length of the ion guide in a second different direction, wherein the second ions are derived from a reagent in accordance with the first aspect of this invention.

In a further aspect there is provided a chemical processing apparatus, including: a fragmentation device, comprising a plurality of electrodes disposed in series along a longitudinal axis of the fragmentation device and defining an ion-manipulation region;
means for applying at least a first DC voltage to at least two contiguous electrodes of the plurality of electrodes to define a first DC potential well, and for applying at least a second DC voltage to at least two different contiguous electrodes of the plurality of electrodes to define a second DC potential well;
means for reducing a DC potential barrier between the first and second DC potential wells to permit positive peptide ions, confined in the first DC potential well, and negatively charged reagent particles, confined in the second DC potential well, to mix; and
a mass-spectrometry module for analyzing at least some fragments of the positive peptide ions extracted from the mixture, wherein the second ions are derived from a reagent in accordance with the first aspect of this invention.

### BRIEF DESCRIPTION OF THE DRAWINGS

The invention is further described by means of example, but not in any limitative sense, with reference to the accompanying drawings, of which:-
FIG. 1a is a block diagram of a chemical processing apparatus in accordance with one embodiment of the invention;
FIG. 1b is a cross-sectional three-dimensional diagram of a fragmentation device that includes stacked ring electrodes in accordance with one embodiment of the invention;
FIG. 2a is a cross sectional schematic view of a stacked-electrode fragmentation device in accordance with one embodiment of the invention;
FIG. 2b is a graph of DC voltages applied to the electrodes shown in FIG. 2a;
FIGS. 3a is a cross sectional schematic view of a stacked-electrode fragmentation device, and an associated graph of the DC voltage as a function of axial position along the fragmentation device, illustrating creating and filling a DC potential well, in accordance with one embodiment of the invention;
FIGS. 3b is a cross sectional schematic view of a stacked-electrode fragmentation device, and an associated graph of the DC voltage as a function of axial position along the fragmentation device, illustrating creating and filling a DC potential well;
FIG. 4 is a schematic view of a fragmentation device and the associated DC voltage graph, illustrating a completed, and filled, positive potential well, and the filling of two neighboring partially formed negative potential wells;
FIG. 5a is a schematic view of a fragmentation device and an associated DC voltage graph, in association with FIG. 4, illustrating a completed, and filled, positive potential well, and completed and filled neighboring negative potential wells;
FIG. 5b is a schematic view of a fragmentation device and an associated DC voltage graph, similar to that of FIG. 5a, in which a positive potential well is filled, a neighboring negative potential well is filled, and a second negative potential well is empty;
FIG. 6a is a schematic view of a device and associated voltage graph, in association with FIGS. 5a and 5b, illustrating an option for mixing and reacting stored opposite-polarity ions, in accordance with one embodiment of the invention;
FIG. 6b is a schematic view of a device and associated voltage graph, illustrating fragments trapped in a single well of a fragmentation device, prior to release from the device;
FIG. 7 is a schematic view of a device and associated voltage graph, illustrating options for releasing biomolecule fragments from the device;
FIG. 8 is a schematic diagram of a chemical processing;
FIG. 9a is a mass spectrum, produced by ETD fragmentation of substance-P (a neuropeptide) using nitrosobenzene as a reagent;
FIG. 9b is a mass spectrum, produced by ETD fragmentation of val⁵-angiotensin using nitrosobenzene as a reagent;
FIG. 9c is a mass spectrum, produced by ETD fragmentation of bradykinin using nitrosobenzene as a reagent.
FIG. 10 is a graphical representation of a theoretical effect of adding various electron withdrawing groups to benzaldehyde.
FIG. 11 is a spectrum showing an example of ETD fragmentation of substance P obtained using 1,4-dicyanobenzene.
FIG. 12 is a spectrum showing an example of ETD fragmentation of substance P obtained using 1,3-dicyanobenzene.
FIG. 13 is a spectrum showing an example of ETD fragmentation of substance P obtained using 1,4- nitrotoluene.

In the drawings, like reference characters generally refer to the same parts throughout the different views. Also, the drawings are not necessarily to scale, emphasis instead generally being placed upon illustrating the principles of the invention.

In the following description, fragments (or products) refer to fragments of biomolecule ions, as formed in stacked-electrode based fragmentation devices of the invention. More generally, multiple types of fragments can occur in LC/MS analyses. In the case of tryptic peptide precursors, fragments can include polypeptide ions that are produced from collisional fragmentation of the intact peptide precursors and whose primary amino acid sequence is contained within the originating precursor peptide.

In some embodiments LC/MS analysis may be carried out. The ions may be fragmented after LC separation and before MS analysis. Such analysis provides an empirical description of a peptide in terms of its mass, charge, retention time, total intensity ion mobility, collision cross section and other data. When a peptide elutes from the chromatographic column, it elutes over a specific retention time period and reaches its maximum signal at a single retention time. After ionization and fragmentation, the peptide appears as a related set of ions. The different ions in the set correspond to different isotopic compositions and charges of the common peptide. Each ion within the related set of ions produces a single peak retention time and peak shape. Since these ions originate from a common peptide, the peak retention time and peak shape of each ion is identical, within a measurement tolerance. Thus, typically, the MS acquisition associated with each peptide produces multiple ion detections for all isotopes and charge states, all sharing the same peak retention-time and peak shape within some measurement tolerance.

Reagent ions, for ETD, may be selected from polyaromatic hydrocarbon compounds. The term "polyaromatic hydrocarbon" (PAH) refers to bi-cyclic or multi-cyclic carbocyclic ring systems including at least two aromatic rings selected from aryl and heteroaryl ring structures. These may include napthalene, fluorene, phenanthrene, pyrene, fluoranthene, chrysene, triphenylene, perylene, acridine; 2,2'-dipyridyl; 2,2'-biquinoline; 9-anthracenecarbonitrile; dibenzothiophene; 1,10'phenanthroline; 9' anthracenecarbonitrile; and anthraquinone. One preferred PAH is fluoranthene.

PAH anions are generated, for example, from low electron-affinity (EA) compounds such as anthracene, 9,10-diphenyl-anthracene, naphthalene, fluorene, phenanthrene, pyrene, fluoranthene, chrysene, triphenylene, perylene, acridine, 2,2'-dipyridyl, 2,2'-biquinoline, 9-anthracenecarbonitrile, dibenzothiophene, 1,10'-phenanthroline, 9' -anthracenecarbonitrile, and anthraquinone. Anions are alternatively generated from substituted derivatives of these low EA compounds. Anions may be also generated from negative electron affinity compounds such as benzene and water clusters, however it is known that the probability for electron transfer is often highest at an EA between 0 and about 400kJ/mol. See, for example, Gunawardena et. al. J. Am. Chem. Soc. 2005, 127, 12627-12639. Furthermore, such anions are generally less stable and more difficult to ionize.

Preferred embodiments of this invention provide reagents for ETD that are not polyaromatic hydrocarbons, some of which include one or more phenyl groups. For example, some azo compounds are suitable. Azobenzene is an effective reagent, though having a relatively high level of toxicity. Suitable reagents have aryl rings having one or more substituents with conjugated double bonds, such as N=N, C=C or c=o.

Some additional related optional reagents include biphenyl, diphenyl ether, hydrazobenzene, 1,3-terphenyl, still bene and phenyl-(4-phenyldiazenylphenyl)diazene.

Preferred embodiments of the invention utilize nitrosobenzene as an ETD reagent. Nitrosobenzene and other single-ring reagents, which have a relatively low mass, **below [ Details] Daltons** would typically be ejected from a quadrupole-based fragmentation device. When employed with a travelling-wave-based fragmentation device, however, some single-ring reagents **which** have the additional advantage of not being ejected.

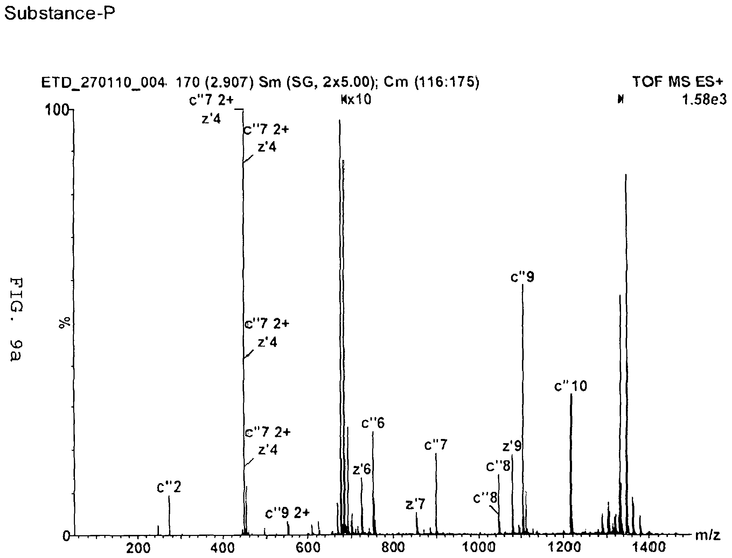

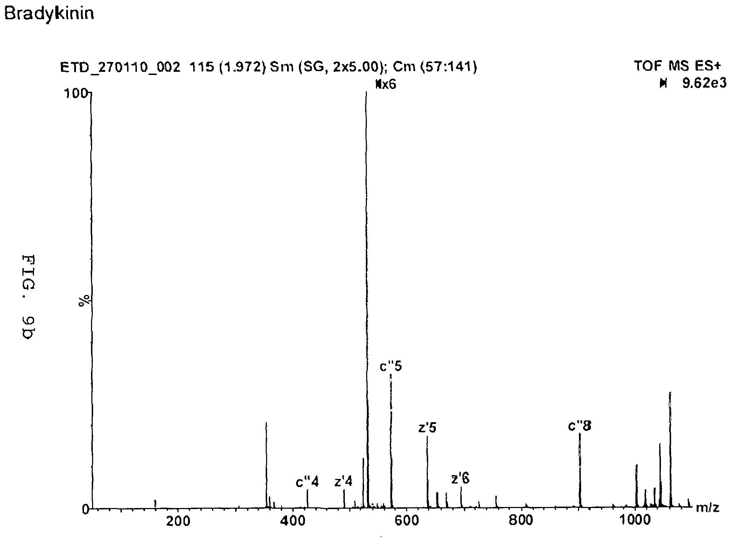

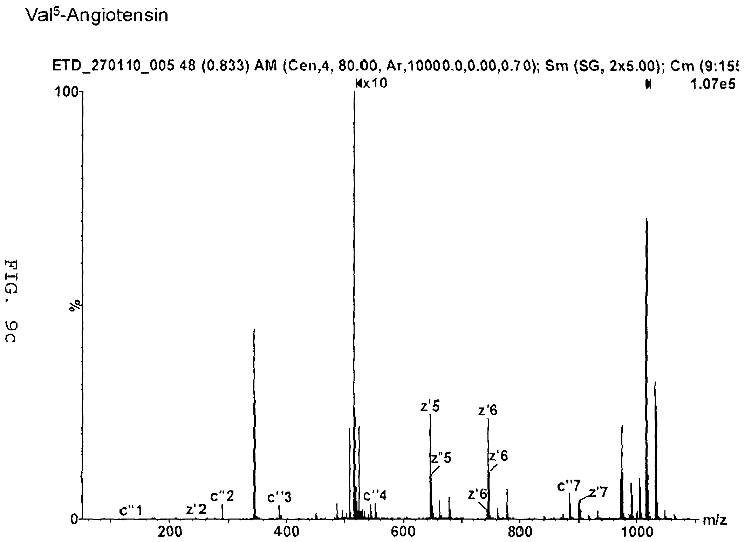

FIGS. 9a, 9b and 9c are mass spectra, which illustrate use of nitrosobenzene as a reagent for analysis of three peptides, using ETD fragmentation (reagent ions having m/z of 107.1.) More generally, some suitable ETD reagents include members of aryl-group compounds, for example single-ring nitroso-substituted aromatics. Preferred reagents comprise phenyl derivatives having a single substituent. Another preferred reagent is iodobenzene.

In a further embodiment of the invention, the reagent comprises a single aromatic ring with one or more substituents. Preferably the substituent should be an electron withdrawing group. Alternatively a substituent may be an electron donating group (EDG) depending on the desired electron affinity.

Substitution of a single benzene nucleus with one or more electron withdrawing groups has the overall effect of increasing the electron affinity of the reagent. This renders the molecule more susceptable to ionisation so that there is a larger anion population produced when a glow discharge source is used for performing ETD.

The Franck-Condon (FC) factor is a measure of the overlap of the vibrational wavefunctions of neutral and anionic species. See Gunawardena et. al. J. Am. Chem. Soc. 2005, 127, 12627-12639.

The preferred range of FC factors for ETD reagents of this invention is between 0.1 and 1.0.

Preferably the molecular structure of the reagent is conjugated.

Preferred embodiments include aryl ketones, including aryl alkyl ketones, for example acetophenone, dibenzylketone and 1,1-diphenylacetone, and aryloxy acetophenones.

Examples of calculated adiabatic electron affinities and FC factors for transitions between the neutral and anion ground state for some of these compounds are shown in Table 1. Calculations were performed using the B3LYP/6-31G(d) protocol. See Foresman, J. B.; Frisch, E. Exploring Chemistry with Electronic Structure Methods, 2nd ed.

**Table 1 - FC factors and adiabatic EA for some monosubstituted benzene derivatives calculated at the level B3LYP/6-31G(d).**

| Reagent | <0\|0>² | EA (kJ/mol) |
|---|---|---|
| benzonitrile | 2.75e-01 | -43.1 |
| benzaldehyde | 2.46e-01 | -15.5 |
| styrene | 2.05e-01 | -87.7 |
| acetophenone | 1.98e-01 | -27.8 |
| nitrobenzene | 1.45e-01 | 51.5 |
| nitrosobenzene | 1.40e-01 | 67.7 |
| ethyl benzoate | 1.01e-01 | -45.4 |
| benzaldehyde oxime | 7.84e-02 | -56.2 |
| anisole | 5.40e-02 | -191.4 |
| aniline | 2.91e-02 | -203.5 |
| ethylbenzene | 8.05e-03 | -188.5 |
| benzamide | 5.73e-03 | -57.8 |
| phenol | 5.08e-03 | -186.9 |
| benzoic acid | 4.48e-03 | -41.2 |
| chlorobenzene | 2.11e-29 | -47.0 |
| bromobenzene | 5.23e-31 | -31.2 |

As is shown in Table 1, many monosubstituted benzene compounds have favourable calculated FC factors but have negative calculated electron affinities. The two compounds with positive electron affinity perform ETD and the use of these reagents constitutes one preferred embodiment of the current invention.

In Table 1 some substituents (for example nitrile, CN) are associated with high FC factors.

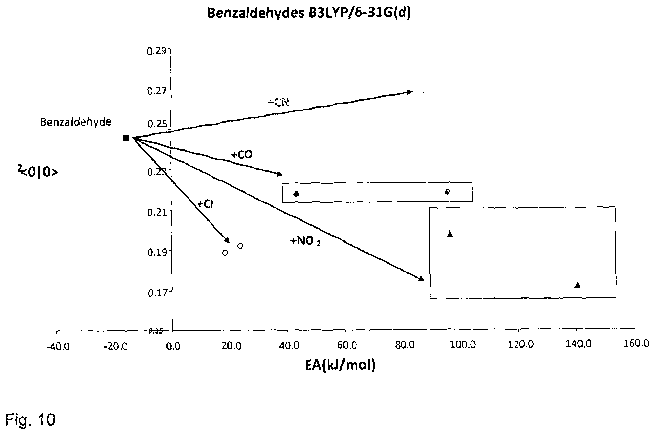

A preferred embodiment of the current invention provides a method for selection of an ETD reagent having the required FC and EA values by choosing one or more substituents with appropriate properties. Figure 10 shows the effect of adding electron withdrawing groups to benzaldehyde, which has a negative calculated electron affinity. In all cases the electron affinity is increased. In most cases the FC factor is reduced with the exception of the addition of the nitrile group CN. This gives the monosubstituted derivative with the highest FC factor (benzonitrile) as shown in Table 1.

**Table 2** shows some disubstitued benzenes with theoretical adiabatic EA and FC factors calculated using the B3LYP/6-31G(d) protocol. The reagent with the highest FC factor is 1,4-dicyanobenzene. The eight reagents with the highest FC factor contain at least one nitrile group.

**Table 2 Disubstitued benzene derivatives with FC factors and adiabatic EA's calculated at the B3LYP/6-31G (d) level.**

| **Reagent** | **<0\|0>²** | **EA (kJ/mol)** |
|---|---|---|
| 1,4-dicyanobenzene | 3.38E-01 | 76.4 |
| 3-cyanostyrene | 3.14E-01 | -2.1 |
| 1,3-dicyanobenzene | 3.00E-01 | 46.5 |
| 3,5-cyanotoluene | 2.82E-01 | 43.1 |
| 4-cyanostyrene (4-ethynylbenzonitrile) | 2.76E-01 | 22.6 |
| 4-formylbenzonitrile | 2.68E-01 | 88.5 |
| 1,4-tolunitrile | 2.59E-01 | -46.2 |
| 2-cyanostyrene | 2.58E-01 | 6.6 |
| 1,3-dinitrobenzene | 2.48E-01 | 139.2 |
| 3,5-nitrotoluene | 2.39E-01 | 134.4 |
| 4-acetobenzonitrile | 2.20E-01 | 74.0 |
| 1,4-benzenedicarboxaldehyde | 2.18E-01 | 95.8 |
| Isopthalaldehyde | 2.17E-01 | 43.3 |
| 3-acetobenzonitrile | 2.14E-01 | 43.1 |
| 3-nitrobenzaldehyde | 1.97E-01 | 96.6 |
| 3-chlorobenzaldehyde | 1.92E-01 | 23.9 |
| 4-chlorobenzaldehyde | 1.88E-01 | 18.6 |
| 1,4-diacetylbenzene | 1.75E-01 | 68.3 |
| 1,2-dicyanobenzene | 1.73E-01 | 57.5 |
| 1,4-dinitrobenzene | 1.71E-01 | 179.3 |
| 4-nitrobenzaldehyde | 1.71E-01 | 140.5 |
| 4-chlorostyrene | 1.67E-01 | -52.0 |
| 1,2-nitrosotoluene | 1.64E-01 | 70.9 |
| 4-chloroacetophenone | 1.59E-01 | 5.8 |
| 3-chloroacetophenone | 1.55E-01 | 10.2 |
| 1,4-nitrotoluene | 1.55E-01 | 46.4 |
| 1,3-nitrosotoluene | 1.35E-01 | 65.6 |
| 2 -acetobenzonitrile | 8.70E-02 | 57.2 |
| 1.3-meta-nitroaniline | 7.34E-02 | 41.7 |
| 1.2-nitrotoluene | 4.45E-02 | 47.0 |
| 1,4-nitroaniline | 4.04E-02 | 18.9 |
| anthranilic acid | 3.35E-02 | -53.8 |
| 4-methoxyaniline | 1.91E-02 | -188.5 |
| 1,2-nitroaniline | 1.69E-02 | 41.5 |
| hydroquinone | 1.03E-03 | -170.9 |

Several of these reagents are efficient ETD reagents including 1,4-dicyanobenzene, 1,3-dicyanobenzene and 1,4-nitrotoluene. These reagents are preferred embodiments of the invention.

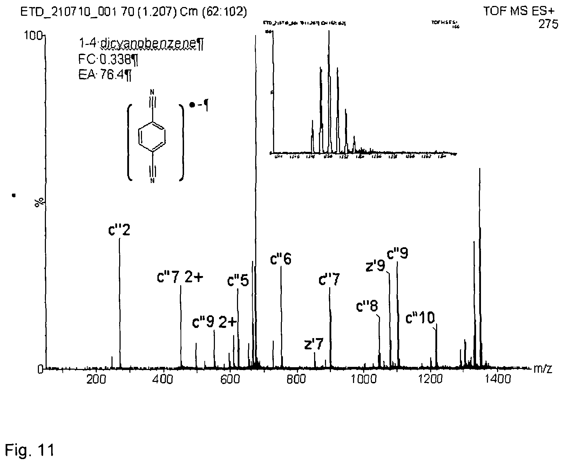

Figure 11 shows the structure of 1,4-dicyanobenzene and an ETD spectrum of substance P obtained using this reagent. The inset spectrum shows the characteristic modification of the singly charged precursor isotope distribution produced by charge reduction of multiply protonated species.

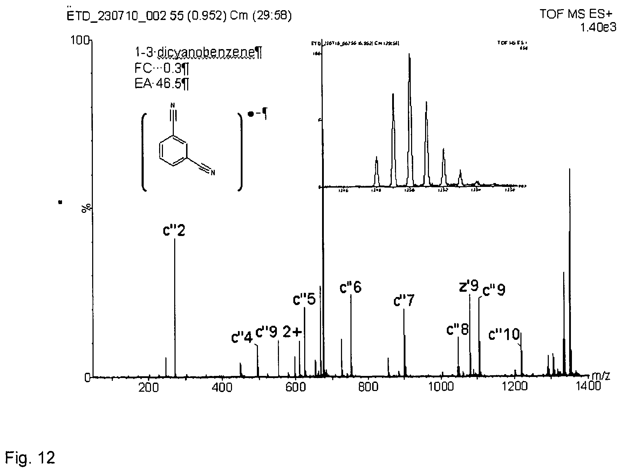

Figure 12 shows the structure of 1,3 dicyanobenzene and an ETD spectrum of substance P obtained using this reagent. The inset spectrum shows the characteristic modification of the singly charged precursor isotope distribution produced by charge reduction of multiply protonated species.

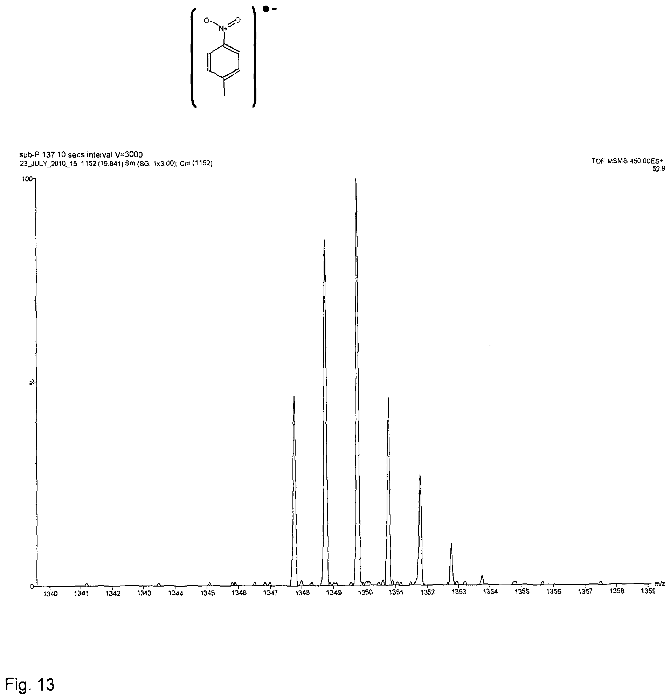

Figure 13 shows the structure of 1,4,-nitrotoluene and an ETD spectrum of substance P obtained using this reagent. The inset spectrum shows the characteristic modification of the singly charged precursor isotope distribution produced by charge reduction of multiply protonated species.

The addition of nitrile groups to aromatic and non-aromatic compounds is beneficial in producing ETD reagents. Preferred examples of non-aromatic nitriles are 1,2-dicyanoethylene and 1,2-dicyanoacetylene.

At the B3LYP/6-31G level of theory, 1,2-icyanoethylene has an EA of 93.5 kJ/mol and an FC factor of 2.66E-1.

Single and multiple derivatisation of the following heteroaromatic compounds are beneficial in generating suitable ETD reagents furan, pyrole, thiophene, imidazole, pyrazole, oxazole, isoxazole, thiozole, triazole.

Preferred derivatives include 5-cyano 1,2,4-triazole, amitrole 2-aminopyridine, 2-pyridine carbonitrile and 3-pyridine carbonitrile. These have favourable FC factors.

Preferred single and multiple substituted derivatives of single-ring compounds include nitriles 3-chlorobenzonitrile; 4-chlorobenzonitrile; 3-pyridinecarbonitrile; and 4-pyridinecarbonitrile.

Sulfur, oxygen and/or nitrogen heterocyclics may be suitable reagent compounds. Substituted azonitrile compounds which may be used include VAZO free-radical source compounds (available from DuPont) such as 4,4'-axobis (4-cyanovaleric acid) sold under the trade mark VAZO 68.

The reagents of this invention may used to generate reagent anions using known techniques, such as atmospheric-pressure chemical ionization (APCI), glow discharge, corona discharge, Townsend discharge, chemical ionization or electrospray ionization (ESI). Some preferred anions have one or more of the following properties:
an m/z range of 20-2000;
are free radical;
are sufficiently stable for transfer into a fragmentation cell; and/or
are double-charged anions.

A preferred embodiment of this invention utilizes a travelling-wave ("T-wave") based device for ETD-type fragmentation. Some embodiments utilize coalescing waves, travelling in one or two directions, as described in WO 2009/066087 and WO 2009/066089, both of which are incorporated herein by reference for all purposes. Coalescing waves (that is, waves that change in magnitude over time, such as decreasing with time and distance as they move through the device) are optionally applied from either end and progress to a mid-region of the device. Preferably, the magnitude of the wave(s) decreases as it progresses, thus delivering ions to a reaction region.

Other embodiments utilize T-wave(s) arranged to move only in one direction or only one direction at any given time. In the latter embodiment negative reagent ions are first loaded into the ETD fragmentation device, and then one or more T-wave(s) maintains movement of positive (analyte) ions through the device. Preferably, the T-wave(s) is tuned to optimize the amount of ETD that occurs. For example, if sample ions remain too long in the device, fragments potentially are neutralized, and may then not be well suited to mass spectrometry, potentially requiring re-ionization.

At each end of the T-wave device, a negative potential is optionally applied to prevent negative ions from escaping, while allowing positive sample ions to pass from the end of the device. The T-wave(s) control motion of positive ions through the device. If enough reagent ions are present, negative barrier potentials are not required to obtain satisfactory ETD. In such a situationa negative DC may be applied at only one end of the fragmentation device, to block reagent ions from leaking out of that end. Use of T-waves from one direction, rather than opposing T-waves, ispreferred.

One or more T-waves may be used to simultaneously transport negative and positive ions through the fragmentation device. Depending on polarity of the wave, negative ions may be moved in front of a wave while positive ions tend to trail; the negative ions "surfing" the "front" of the wave while the positive ions ride the "crest" of the wave (viewed as a trough from the polarity perspective of the positive ions.)

The preferred amount of time required for an ETD reaction is in a range of a few milliseconds to approximately 100 milliseconds. A reaction time may be selected by balancing an increase in time (to permit more or sufficient time for negative and positive ions to mix and react) with a decrease in time (to avoid eventual neutralization of fragments.) Thus, for example, ions are admitted into the device, and then guided out of the device after a selected amount of time; optionally, the T-wave(s) maintains axial movement of ions along the device for part or all of their time spent in the device.

A variety of rod-based, stacked-electrode and other ion-controlling electrode configurations are suitable bases for an ETD fragmentation device. Merely for illustrative purposes, the following describes some approaches to implementing ETD in stacked-electrode-based devices. These examples are not intended to limit the range of application of the invention.

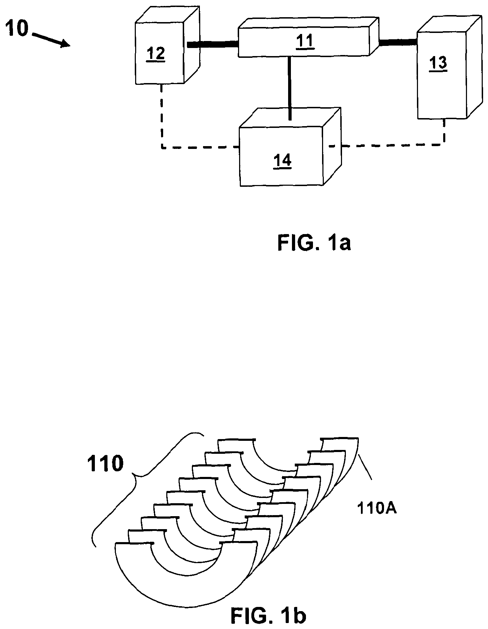

FIG. 1a is a block diagram of chemical processing apparatus (10) according to one embodiment of the invention. The apparatus includes a fragmentation device (11) and a control unit (14), and includes an ion supply module (12) and a mass-spectrometry module (13). The fragmentation device (11) includes a linear arrangement of electrodes, herein referred to as "stacked electrodes". The stacked electrodes define a curved path through the device (11), but the device (11) would still be viewed herein as being "linear". The control unit (14) controls the application of voltages to the electrodes of the fragmentation device (11).

FIG. 1b is a cross-sectional three-dimensional diagram of one embodiment of the fragmentation device (11). The device (11), in this example, includes a set (110) of stacked ring electrodes (110A), in a linear arrangement. Each ring electrode (110A) has a central aperture. The co-linear arrangement of apertures defines an ion radial-confinement region and a longitudinal direction of the fragmentation device (11). A perpendicular direction, relative to the longitudinal direction, is herein referred to as a "radial" direction. The longitudinal direction is also referred to herein as the axial direction.

The apertures, in this example, are depicted as having a circular shape. Though preferred, the particular shape and/or size of the apertures may be optionally varied, as desired. The apertures also define an ion-manipulation region and a reaction region, within the device (11).

As an illustrative example, the stacked electrodes (110A) may be composed of conductive circular stainless steel ring plates with a pitch of 1.5 mm, a thickness of 0.5 mm and a central aperture diameter of 5 mm. An argon buffer gas pressure of 0.076 Torr is optionally used. The length of the device is optionally 90 mm. The control unit (14) applies opposing phases of 100 V RF to adjacent electrodes (110A) to provide radial confinement of positive and negative ions.

The use of fragmentation devices for mutual confinement of opposite polarity particles is described with reference to the fragmentation device (110) having stacked ring electrodes (110A). In view of the following description, however, it will be clear that principles of the invention are applicable to a variety of configurations of stacked electrodes.

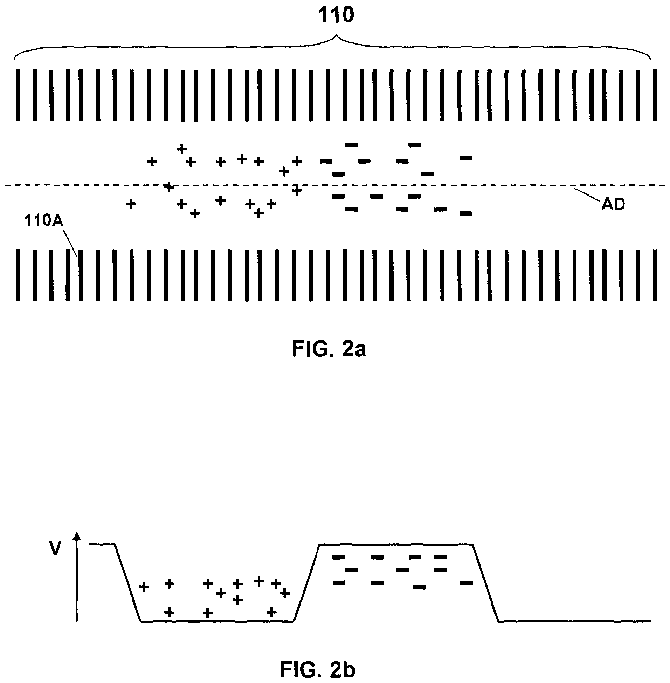

FIG. 2a is a cross sectional schematic view of the set (110) of stacked ring electrodes (110A), under a particular operating condition, illustrating some general principles of one embodiment of the invention. The dashed line indicates the longitudinal axis AD, associated with the axial direction, of the device. The electrodes (110A) contain positively charged biomolecule ions and negatively charged particles, such as reagent ions or electrons. The ions and particles are trapped in neighboring potential wells (see FIG. 2b.) The wells are formed through appropriate application, by the control unit (14), of DC voltages to the individual ring electrodes (110A).

FIG. 2b, in association with FIG. 2a, is a qualitative graph of the DC potential along the device (11), as produced by applying DC voltages to the electrodes (110A) (the confined biomolecule ions and charged particles are illustrated, for comparison to FIG. 2a.) The control unit (14) is configured to controllably and variably apply similar or different DC voltage potentials to the electrodes (110A) to form negative or positive potential wells, of various length, depth and duration to separately and simultaneously confine negatively and positively charge particles in the fragmentation device (11). As illustrated in FIGS. 2a and 2b, a negative potential well has been created by applying a series of negative DC voltages to several adjacent electrodes (110A) and, similarly, a positive potential well has been created by applying a series of positive DC voltages to a neighboring group of adjacent electrodes (110A).

In FIG. 2b, the biomolecule ions and the negative particles are illustrated as partially filling their associated wells at different energy levels. Ions with greater kinetic energy are depicted as residing nearer to the top of the associated well. Well depths are often chosen to be greater than an ion maximum kinetic energy level, to prevent ions from escaping from the well.

Positive and negative particles are optionally confined in one or more wells, each well associated with one or more electrodes (110A) having an applied DC voltage that islower or higher than a neighboring electrode.

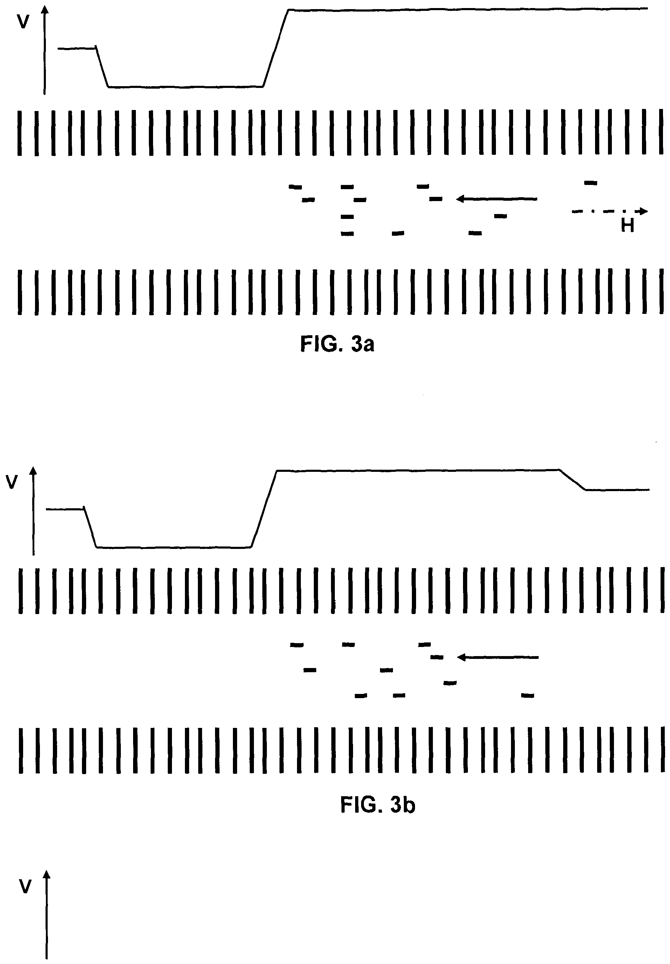

Next, referring to FIGS. 3a to FIG. 5, some methods are described of filling and forming DC potential wells, in apparatus of the invention. Filling and forming of DC potential wells is accomplished in any suitable manner. A well is optionally partially formed prior to filling.

Illustrating one option for filling a potential well, FIGS. 3a and 3b each are cross sectional schematic representations of the stacked-electrode fragmentation device (11), depicting the electrodes (110A) and an associated qualitative graph of the DC voltage as a function of position along the set (110) of electrodes (110A). The location of biomolecule ions and negatively charged particles is indicated. Plus signs denote positively charged biomolecule ions and negative signs denote negatively charged reagent ions and/or free electrons. A control module (14), is configured to apply the DC voltages to each electrode (110A) to support formation and filling of DC potential wells.

In FIG. 3a, a DC potential barrier has been formed, while admitting negatively charged particles (reagent ions and/or free electrons), so that the ions, admitted at one end of the set (110) do not flow through the set (110) and out the other end. After a desired quantity of particles have been admitted into the fragmentation device (11), the control module adjusts DC voltages applied to the electrodes (110A) to form a second DC barrier, preventing the admitted negatively charged particles from escaping, i.e., completing formation of a DC potential well.

A magnetic field generator, such as a permanent magnet or an electromagnet may be used. A magnetic field (field direction indicated by arrow H) is then optionally disposed in the radial-confinement region of the electrodes (110A). The magnetic field supports radial confinement of electrons, assisting optional use of the device (11) forECD.

FIG. 3b illustrates a variation of the DC voltage configuration depicted in FIG. 3a. The negatively charged particles are admitted into a partially formed well. It is preferred to complete the well after admitting ions and/or electrons to reduce the kinetic energy that the ions/electrons have upon confinement in the well. Collisional cooling with a buffer gas, such as argon may serve to reduce the kinetic energy of ions in the fragmentation device (11).

Negatively charged reagent ions may be provided by any suitable source, including known sources, such as a chemical-ionization (CI) source.

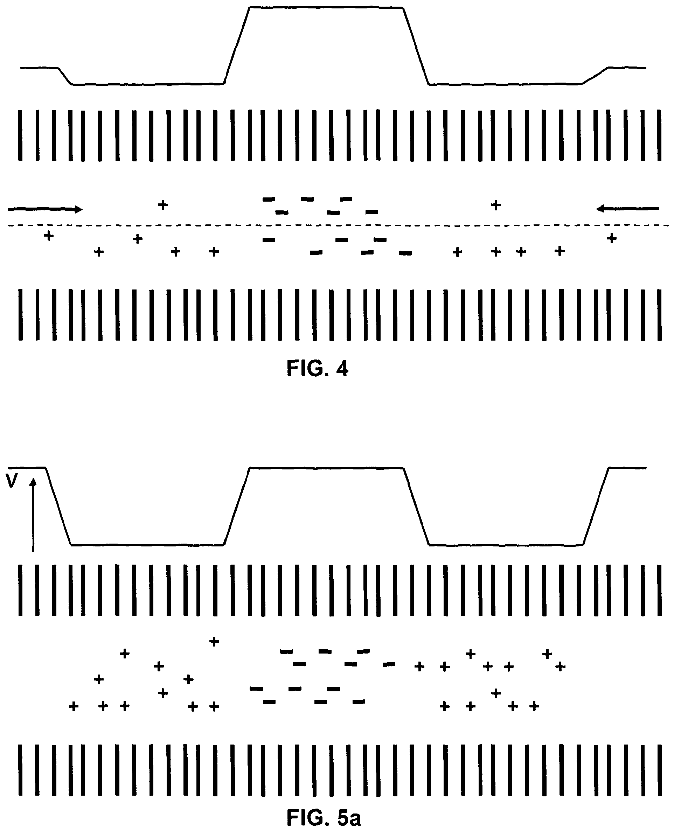

FIG. 4 illustrates a completed, and filled, positive potential well, and the filling of two neighboring partially formed negative potential wells. Positive biomolecule ions are admittedfrom both ends of the fragmentation device (11). Wells may be optionally formed and filled in a variety of sequences, with biomolecule ions and reagent ions being admitted from one or both ends of the set (110) of stacked electrodes (110A). In one preferred embodiment, three filled wells are created as follows:
1) a positive DC potential barrier is formed and biomolecule ions are admitted from one end of the device (11);
2) the first well is completed by forming a second DC potential barrier to axially confine the admitted biomolecule ions;
3) negative reagent ions are admitted from the same end of the device as were the biomolecule ions (the reagent ions encounter a negative potential barrier that maintains their separation from, and adjacent to, the stored biomolecule ions;)
4) the second well is completed by forming a third DC potential barrier to axially confine the admitted reagent ions;
5) a third filled well (for more biomolecule ions) is created in a manner similar to the first well. At this stage of processing the device (11) appears as illustrated by FIG. 5a.

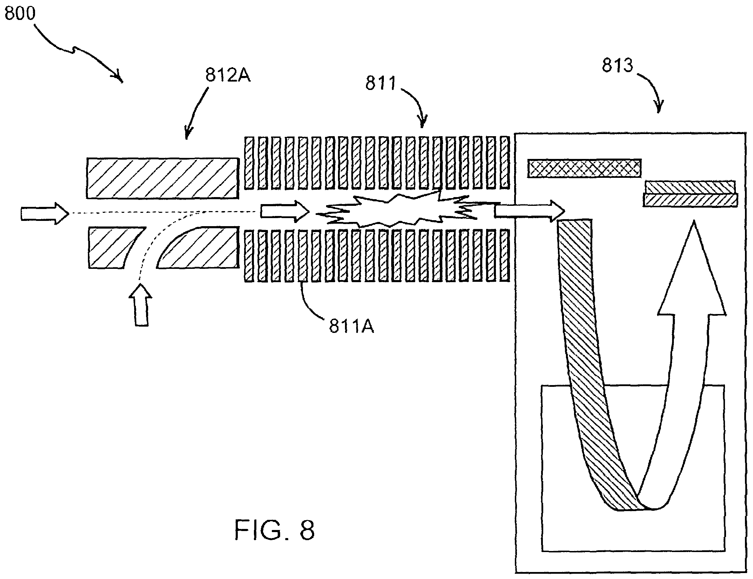

Suitable biomolecule ions include, for example, singly- or multiply-protonated peptide molecules (precursor ions), generated by a ionized-sample source. The source (12), may be any suitable source. For example, the biomolecule ion source (12) may be any one of, or combination of:
(i) an electrospray ionization (ESI) ion source;
(ii) (ii) an atmospheric pressure;
(iii) (iii) an atmospheric pressure chemical ionization (("APCI") ion source;
(iv) (iv) an atmospheric pressure photo ionization ("AAPPI") ion source;
(v) (v) a matrix assisted laser desorption ionization ("MALDI") ion source;
(vi) (vi) a laser desorption ionization ("LDI") ion source;
(vii) (vii) an inductively coupled plasma ("ICP) ion source;
(viii) (viii) an electron impact ("EI") ion source;
(ix) (ix) a chemical ionization ("CI") ion source;
(x) (x) a fast atom bombardment ("FAB") ion source; and
(xi) (xi) a liquid secondary ions mass spectrometry ("LSIMS") ion source. Further alternatives for a source (12) are described below, with reference to FIG. 8.

As an alternative to electron transfer, protons may be transferred from one or more multiply charged analyte cations or positively charged ions to one or more neutral, non-ionic or uncharged reagent gases or vapors whereupon at least some of the multiply charged analyte cations or positively charged ions are reduced in charge state. It is also contemplated that some cations may be induced to dissociate and form product or fragment ions. The multiply charged analyte cations preferably include peptides, polypeptides, proteins or other biomolecules.

FIG. 5a illustrates three completed, and filled, DC potential wells: a positive potential well storing reagent ions and two neighboring negative potential wells storing positively charged biomolecule ions. It will be apparent that one or more wells are optionally formed and filled for each polarity of particles, with particles admitted from one or both ends of a device.

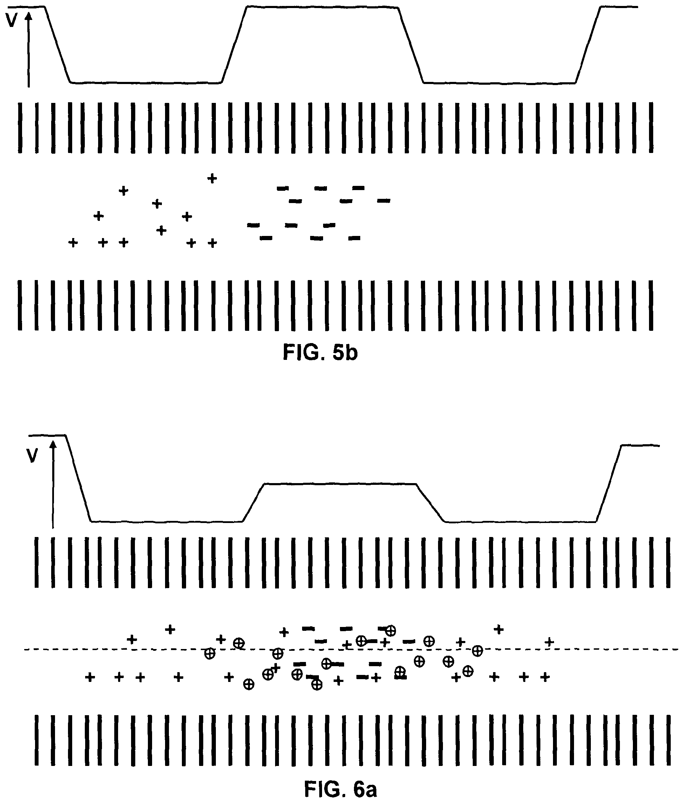

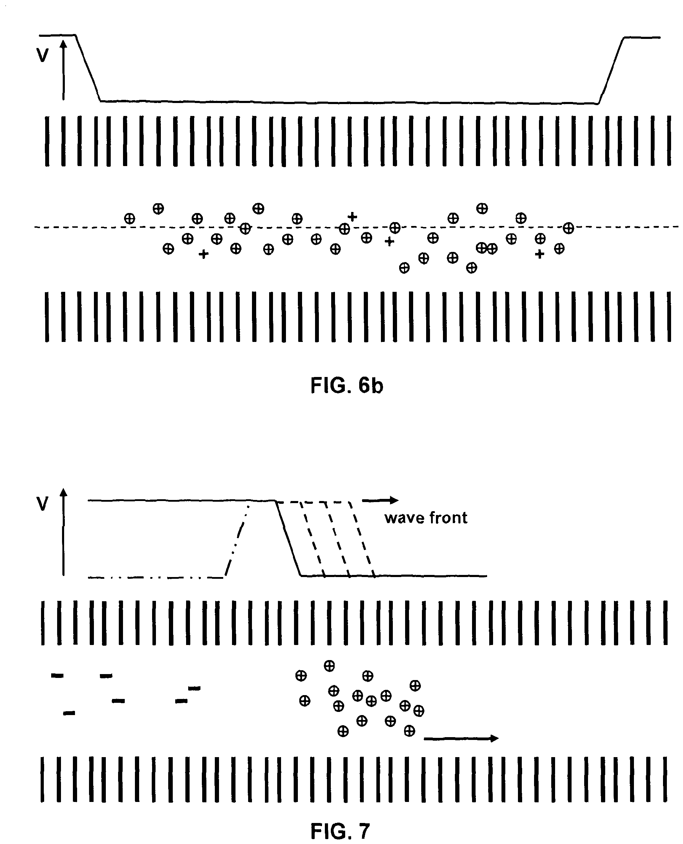

FIG. 5b is a schematic view of a device and an associated DC voltage graph, similar to that of FIG. 5a, in which a positive potential well is filled, a neighboring negative potential well is filled, and a second negative potential well is empty;
, The formation and ejection of biomolecule fragments are described with reference to FIGS. 6a, 6b and 7.

FIG. 6a, in association with FIG. 5a, illustrates one option for mixing of stored opposite-polarity ions. The nested reagent ion well has been reduced in depth, to commence mixing The DC voltage level applied to the electrodes (110A) defining the negative potential well has been reduced.

The reduction in well depth is associated with a corresponding height reduction of the two DC potential barriers associated with the well. The barrier reduction permits biomolecule ions to react with the negatively charged ions. By preserving some level of the negative well, at least some confinement of negative ions remains.

The reduction in well depth is particularly suitable ifat least some of the biomolecule ions have greater kinetic energy than at least some of the regent particles. In this case, the reduced well can continue to confine negative particles while admitting biomolecule ions. Room temperature electrons can be confined in a reduced well, while hotter biomolecule ions can penetrate the remaining potential barrier associated with the reduced well, to react with the confined electrons.

Alternatively, the nested negative potential well is entirely removed (see FIG. 6b,) leaving a single, large positive well. In this case, some negative ions will flow past the positive biomolecule ions, and out of the reaction zone in the stacked electrodes (110A).

As the oppositely charged ions mix and react, at least some of the biomolecule ions fragment, as illustrated in FIGS. 6a and 6b. FIG. 6b illustrates fragments trapped in a single well of a fragmentation device, prior to release from the device.

The fragments may be manipulated and/or analyzed in the fragmentation device and/or ejected from the fragmentation device for analysis in the mass-spectrometry unit (13).

Where the negatively charged particles are electrons, the invention provides ECD of peptides. Capture of an electron by a protonated peptide is associated with an exothermic reaction, releasing, for example, 6 eV. The peptide's backbone fragments, leading, for example, to c and z fragments.

Next, referring to FIG. 7, one alternative to allowing ions to flow out of the fragmentation device (110) is to use travelling DC potential barriers to propel ions toward an exit of the device and/or separate ions prior to ejection. See, e.g., Giles et al., "Applications of a Travelling Wave-Based Radio-Frequency-Only Stacked Ring Ion Guide", Rapid Commun. in Mass Spectrom., 2004, 2401-2414.

FIG. 7 illustrates two options for propelling peptide fragments from the fragmentation device (11). A DC potential barrier (as shown with dashed line) or a DC potential pulse (as shown with variable dashed line) travels toward one end of the set (110) stacked rings (110A). The fragments "surf' on the barrier, or pulse, until reaching an end of the set (110). At the same time, remaining negatively charged reagent ions are accelerated, crossing the barrier, toward the opposite end of the set (110). As is known in the mass analysis arts, a series of travelling pulses can be used to separate ions according to their mobility.

Mobility separation is optionally used, for example, to remove ions having a mass outside a selected mass window around precursor ions and/or reagent ions. Ion separation can improve mass-analysis sensitivity and duty cycle.

As noted, the apparatus (10) optionally includes an analysis module (13). In one alternative embodiment, the fragments are fed to a mass spectrometer (MS). The MS may be any suitable device. The MS may be a quadrupole mass filter, a time-of-flight (TOF) mass analyzer, a Fourier Transform Ion Cyclotron Resonance (FTICR) mass analyzer, or a 2D (linear) quadrupole ion trap or a 3D (Paul) quadrupole ion trap.

Some embodiments of the invention include separate ion sources for analytes and regents, to generate cation analytes and reagent anions. In order to efficiently introduce both cations and anions from separate ion sources into a fragmentation device (11), one option is to use an ion guide that simultaneously and continuously receives and transfers ions of either polarity from multiple ion sources at different locations. A suitable ion guide is described in U.S. Patent No. 6,891,157 to Bateman.

FIG. 8 is a schematic diagram of an chemical analysis apparatus (800). The apparatus (800) includes an ion guide (812A), a fragmentation device (811), and a TOF mass analyzer (813). The ion guide (812A) introduces both cations and anions, from separate ion sources, into the fragmentation device (811).The TOF mass analyzer (813) receives peptide ions and ion fragments from the fragmentation device (811). The fragmentation device (811) includes stacked electrodes (811A). It is preferable that a mass selective device, such as a quadrupole-based mass filter, is utilized to select the analyte and/or the reagent between each ion source and the ion guide (812A). The TOF mass analyzer (813) analyzes the product ions downstream of the reaction zone of the fragmentation device (811).

The control unit (14) may have any suitable configuration for individually and selectively applying DC voltages to the electrodes (110A) of the fragmentation device (11). The control unit (14) is in data and/or electrical communication with other components of the apparatus (10) via wired and/or wireless means. The control unit (14) receives process data from the mass-spectrometer module (13), and provides control signals to other components, for example, the fragmentation device (11). The control unit (14) is configured to support automation of operation of the apparatus (10). The control unit (14), may be implemented in software, firmware, and/or hardware (e.g., as an application-specific integrated circuit), and may include a user interface. The control unit (14) includes and/or is in communication with storage component(s).

Suitable implementations of the control unit (14) include, one or more microprocessors. A single integrated circuit or microprocessor includes the control unit (14) and other electronic portions of the apparatus (10). One or more microprocessors implement software that enables the functions of the control unit (14). In some embodiments, the software is designed to run on general-purpose equipment and/or specialized processors dedicated to the functionality herein described.

In some implementations of the apparatus (10), the control unit (14) includes a user interface to support interaction with the control unit (14) and/or other portions of the apparatus (10). For example, the interface is configured to accept control information from a user and to provide information about the apparatus (10) to a user. The user interface is usedto set system control parameters and/or to provide diagnostic and troubleshooting information to the user. In one embodiment the user interface provides networked communication between the apparatus (10) and users located either local to the operating environment or remote from the operating environment. The user interface in some implementations is used to modify and update software.

Where a travelling pulse and/or barrier is used to manipulate ions in the fragmentation device (11), the control unit (14) is configured to control parameters such as pulse shape, wavelength and amplitude. These parameters can also be optimized to provide control over the relative ion velocity of cations and anions. The velocity of ion-neutral collisions can be manipulated to exploit collision induced dissociation (CID) within the fragmentation device (13).

Variations, modifications and other implementations of what is described herein will occur to those of ordinary skill in the art without departing from the scope of the invention as claimed.

## Claims

1. Use of a compound as a mass spectrometry electron transfer dissociation reagent, wherein the compound is a substituted aromatic compound having a Franck-Condon factor of between 0.1 and 1.0 and an electron affinity having a positive value between 0.1 to 200 kJ/mol, and wherein either the aromatic nucleus is substituted by one or more substituents consisting of cyano, nitroso, carboxyl, iodo, aldehyde and acetoxy moieties, or the compound is 1,4-nitrotoluene.

2. A use as claimed in claim 1, wherein the one or more substituents is cyano.

3. A use as claimed in claim 1 or 2, wherein the reagent has an electron affinity with a positive value between 0.1 to 150 kJ/mol, preferably 0.1 to 100 kJ/mol.

4. A use as claimed in any preceding claim, wherein the aromatic nucleus is selected from the group consisting of phenyl, pyridinyl, pyrimidinyl, triazolyl, styrenyl and picolinyl.

5. A use as claimed in any preceding claim wherein the compound is selected from the group consisting of
1,4-dicyanobenzene
1,3-dicyanobenzene
1-4-nitrotoluene
1,3-benzenedicarboxaldehyde (isopthalaldehyde)
1,2 ortho-nitrosotoluene
benzaldehyde oxime
nitrosobenzene
3-pyridinecarbonitrile
ethyl cis (beta cyano) acrylate
1,2,4,5-cyanobenzene
1,4-benzenedicarboxaldehyde (terephthalaldehyde)
3 acetobenzonitrile
4-formyl benzonitrile

6. A use as claimed in any preceding claim wherein the compound is selected from the group consisting of
1,2-dicyanoethylene
5-cyano-1,2,4-triazole (124-triazole-5-carbonitrile)
2,5-dicyanopyrimidine
3,5-dicyanopyridine
5-methylpyridine 3-carbonitrile
5-pyrimidinecarbonitrile
2,4-dicyanopyrimidine
3,5-dicyanotoluene
4-cyanostyrene (4-ethynylbenzonitrile)
benzaldehyde
2-cyanostyrene
1,3,4,5-cyanobenzene
3-methyl picolinonitrile
4-vinylpyridine
4-pyrimidine carbonitrile
2-pyrimidine carbonitrile
4-acetobenzonitrile
2-pyrazinecarbonitrile
3-acetopyridine
4-cyanonitrobenzene
3-chlorobenzaldehyde
3-cyanonitrobenzene
1,4-diacetylbenzene
1,2-dicyanobenzene
3-nitrosopyridine
4-acetopyridine
4-chloroacetophenone
3-chloroacetophenone
3,5-dichlorobenzonitrile
1,3-meta-nitrosotoluene
1,2,3-tricyanobenzene
3,5-chloroacetophenone
4-nitrosopyridine
3-amino-4-cyanopyridine
2-amino-4-cyano pyridine
2-acetopyridine
2-nitrosopyridine
4-amino-2-cyanopyridine
2-acetobenzonitrile
4-amino-3-cyanopyridine
1,3-cyclohexanedione
4-amino-5-pyrimidine carbonitrile
4,5-dicyanoimidazole
3-thiophene carbonitrile
2-cyanoethyl acrylate
diaminomaleonitrile
2-cyanothioacetamide
phthalic acid
4-chloropyridine
2,4,6-CHT-36-carbonitrile
acetyl malonitrile
ethyl-2-cyanoacrylate
2-cyano-3-methyl but-2-enoic acid
1,2-dicyanoethyne (acetylene dinitrile)

7. A use as claimed in any of claims 1 to 6 wherein the compound forms a radical ion in use.

8. A method of characterisation of an analyte comprising the steps of providing a first supply of analyte cations using electrospray ionization;
providing a second supply of reagent anions using an ionization source;
combining the first and second supplies; and
carrying out a mass spectrometry (MS) technique on the combined supplies;
wherein the reagent is a substituted aromatic compound having a Franck-Condon factor of between 0.1 and 1.0 and an electron affinity having a positive value between 0.1 to 200 kJ/mol, and wherein either the aromatic compound is substituted by one or more substituents consisting of cyano, nitroso, carboxyl, iodo, aldehyde and acetoxy moieties,
or wherein the compound is 1,4-nitrotoluene.

9. A method as claimed in claim 8 wherein the analyte is a biomolecule.

10. A method as claimed in claim 9 wherein the analyte is a polypeptide.

11. A method as claimed in any of claims 8 to 10, including the step of using an electron transfer dissociation device, adapted so that: (a) in a mode of operation one or more first transient DC voltages or potentials or one or more first transient DC voltage or potential waveforms are applied to at least some of a plurality of electrodes in order to drive or urge at least some product or fragment ions along at least a portion of the axial length of the electrodes in a direction different to a first direction; and/or (b) in a mode of operation the one or more second transient DC voltage or potentials or one or more second transient DC voltage or potential waveforms are applied to at least some of the plurality of electrodes in order to drive product or fragment ions along and/or through at least a portion of the axial length of the electrodes in a direction different to the second direction.

12. A method as claimed in any of claims 8 to 11 including the steps of:
providing an electron transfer dissociation device including a plurality of stacked electrodes;
applying one or more first transient DC voltages or potentials or one or more first transient DC voltage or potential waveforms to at least some of the plurality of electrodes in order to drive at least some first ions along at least a portion of the axial length of the ion guide in a first direction; and applying one or more second transient DC voltages or potentials or one or more second transient DC voltage or potential waveforms to at least some of the plurality of electrodes in order to drive at least some second ions along at least a portion of the axial length of the ion guide in a second different direction, wherein the second ions are derived from said reagent.

13. A method as claimed in any of claims 8 to 12, including the step of providing:
a fragmentation device, comprising a plurality of electrodes disposed in series along a longitudinal axis of the fragmentation device and defining an ion-manipulation region;
means for applying at least a first DC voltage to at least two contiguous electrodes of the plurality of electrodes to define a first DC potential well, and for applying at least a second DC voltage to at least two different contiguous electrodes of the plurality of electrodes to define a second DC potential well;
means for reducing a DC potential barrier between the first and second DC potential wells to permit positive peptide ions, confined in the first DC potential well, and negatively charged reagent particles, confined in the second DC potential well, to mix; and
a mass-spectrometry module for analyzing at least some fragments of the positive peptide ions extracted from the mixture, wherein the second ions are derived from said reagent

## Patentansprüche

1. Verwendung einer Verbindung als Massenspektrometrie-Elektronentransfer-Dissoziationsreagens, wobei die Verbindung eine substituierte aromatische Verbindung mit einem Franck-Condon-Faktor zwischen 0,1 und 1,0 und einer Elektronenaffinität mit einem positiven Wert zwischen 0,1 und 200 kJ/mol ist und wobei entweder der aromatische Kern mit einem oder mehreren Substituenten substituiert ist, die aus Cyano-, Nitroso-, Carboxyl-, Iod-, Aldehyd- und Acetoxyeinheiten bestehen, oder die Verbindung 1,4-Nitrotoluol ist.

2. Verwendung nach Anspruch 1, wobei der eine oder die mehreren Substituenten Cyano ist bzw. sind.

3. Verwendung nach Anspruch 1 oder 2, wobei das Reagens eine Elektronenaffinität mit einem positiven Wert zwischen 0,1 und 150 kJ/mol, vorzugsweise 0,1 bis 100 kJ/mol, ist.

4. Verwendung nach einem vorstehenden Anspruch, wobei der aromatische Kern aus der Gruppe ausgewählt ist, bestehend aus Phenyl, Pyridinyl, Pyrimidinyl, Triazolyl, Styrenyl und Picolinyl.

5. Verwendung nach einem vorstehenden Anspruch, wobei die Verbindung aus der Gruppe ausgewählt ist, bestehend aus:
1,4-Dicyanobenzol
1,3-Dicyanobenzol
1,4-Nitrotoluol
1,3-Benzoldicarboxaldehyd (Isophthalaldehyd)
1,2-Ortho-nitrosotoluol
Benzaldehydoxim
Nitrosobenzol
3-Pyridincarbonitril
Ethyl-cis-(betacyano)acrylat
1,2,4,5-Cyanobenzol
1,4-Benzoldicarboxaldehyd (Terephthalaldehyd)
3-Acetobenzonitril
4-Formylbenzonitril

6. Verwendung nach einem vorstehenden Anspruch, wobei die Verbindung aus der Gruppe ausgewählt ist, bestehend aus:
1,2-Dicyanoethylen
5-Cyano-1,2,4-triazol (124-Triazol-5-carbonitril)
2,5-Dicyanopyrimidin
3,5-Dicyanopyridin
5-Methylpyridin-3-carbonitril
5-Pyrimidincarbonitril
2,4-Dicyanopyrimidin
3,5-Dicyanotoluol
4-Cyanostyrol (4-Ethynylbenzonitril)
Benzaldehyd
2-Cyanostyrol
1,3,4,5-Cyanobenzol
3-Methylpicolinonitril
4-Vinylpyridin
4-Pyrimidincarbonitril
2-Pyrimidincarbonitril
4-Acetobenzonitril
2-Pyrazincarbonitril
3-Acetopyridin
4-Cyanonitrobenzol
3-Chlorbenzaldehyd
3-Cyanonitrobenzol
1,4-Diacetylbenzol
1,2-Dicyanobenzol
3-Nitrosopyridin
4-Acetopyridin
4-Chloracetophenon
3-Chloracetophenon
3,5-Dichlorbenzonitril
1,3-meta-Nitrosotoluol
1,2,3-Tricyanobenzol
3,5-Chloracetophenon
4-Nitrosopyridin
3-Amino-4-cyanopyridin
2-Amino-4-cyanopyridin
2-Acetopyridin
2-Nitrosopyridin
4-Amino-2-cyanopyridin
2-Acetobenzonitril
4-Amino-3-cyanopyridin
1,3-Cyclohexandion
4-Amino-5-pyrimidincarbonitril
4,5-Dicyanoimidazol
3-Thiophencarbonitril
2-Cyanoethylacrylat
Diaminomaleonitril
2-Cyanothioacetamid
Phthalsäure
4-Chlorpyridin
2,4,6-CHT-36-carbonitril
Acetylmalonitril
Ethyl-2-cyanoacrylat
2-Cyano-3-methylbut-2-ensäure
1,2-Dicyanoethyn (Acetylendinitril)

7. Verwendung nach einem der Ansprüche 1 bis 6, wobei die Verbindung in Gebrauch ein Radikalion bildet.

8. Verfahren zum Charakterisieren eines Analyten, umfassend die Schritte des Bereitstellens einer ersten Speisung von Analytkationen unter Verwendung von Elektrosprayionisation;
Bereitstellens einer zweiten Speisung von Reagensanionen unter Verwendung einer Ionisationsquelle;
Kombinierens der ersten und der zweiten Speisung; und Durchführens einer Massenspektrometrie-(MS-)Technik an den kombinierten Speisungen;
wobei das Reagens eine substituierte aromatische Verbindung mit einem Franck-Condon-Faktor zwischen 0,1 und 1,0 und einer Elektronenaffinität mit einem positiven Wert zwischen 0,1 und 200 kJ/mol ist und wobei entweder die aromatische Verbindung mit einem oder mehreren Substituenten substituiert ist, die aus Cyano-, Nitroso-, Carboxyl-, Iod-, Aldehyd- und Acetoxyeinheiten bestehen, oder die Verbindung 1,4-Nitrotoluol ist.

9. Verfahren nach Anspruch 8, wobei der Analyt ein Biomolekül ist.

10. Verfahren nach Anspruch 9, wobei der Analyt ein Polypeptid ist.

11. Verfahren nach einem der Ansprüche 8 bis 10, das den Schritt des Verwendens einer Elektronentransferdissoziationsvorrichtung beinhaltet, die so ausgelegt ist, dass: (a) in einem Betriebsmodus ein oder mehrere erste transiente DC-Spannungen oder -Potentiale oder eine oder mehrere erste transiente DC-Spannungs- oder -Potentialwellenformen an zumindest manche einer Mehrzahl von Elektroden angelegt werden, um zumindest manche Produkt- oder Fragmentionen entlang zumindest eines Abschnitts der Axiallänge der Elektroden in einer anderen Richtung als eine erste Richtung zu treiben oder zu drängen; und/oder (b) in einem Betriebsmodus die eine oder mehreren zweiten transienten DC-Spannung oder -Potentiale oder einen oder mehreren zweiten transienten DC-Spannungs- oder -Potentialwellenformen an zumindest manche der Mehrzahl von Elektroden angelegt werden, um Produkt- oder Fragmentionen entlang und/oder durch zumindest einen Abschnitt der Axiallänge der Elektroden in einer anderen Richtung als die zweite Richtung zu treiben oder zu drängen.

12. Verfahren nach einem der Ansprüche 8 bis 11, das die Schritte beinhaltet:
Bereitstellen einer Elektronentransferdissoziationsvorrichtung, die eine Mehrzahl von gestapelten Elektroden beinhaltet;
Anlegen einer oder mehrerer erster transienter DC-Spannungen oder -Potentiale oder einer oder mehrerer erster transienter DC-Spannungs- oder -Potentialwellenformen an zumindest manche erste Ionen entlang zumindest eines Abschnitts der Axiallänge des Ionenleiters in einer ersten Richtung zu treiben oder zu drängen; und/oder Anlegen einer oder mehrerer zweiter transienter DC-Spannungen oder - Potentiale oder einer oder mehrerer zweiter transienter DC-Spannungs- oder -Potentialwellenformen an zumindest manche der Mehrzahl von Elektroden, um zumindest manche zweite Ionen entlang zumindest eines Abschnitts der Axiallänge des Ionenleiters in einer zweiten anderen Richtung zu treiben oder zu drängen, wobei die zweiten Ionen vom Reagens abgeleitet werden.

13. Verfahren nach einem der Ansprüche 8 bis 12, das die Schritte des Bereitstellens beinhaltet:
einer Fragmentierungsvorrichtung, die eine Mehrzahl von Elektroden umfasst, die entlang einer Längsachse der Fragmentierungsvorrichtung in Reihe angeordnet sind, und des Definierens einer Ionenmanipulationsregion;
eines Mittels zum Anlegen zumindest einer ersten DC-Spannung an zumindest zwei zusammenhängende Elektroden der Mehrzahl von Elektroden, um einen ersten DC-Potentialtopf zu definieren, und zum Anlegen zumindest einer zweiten DC-Spannung an zumindest zwei verschiedene zusammenhängende Elektroden der Mehrzahl von Elektroden, um einen zweiten DC-Potentialtopf zu definieren;
eines Mittels zum Verringern einer DC-Potentialbarriere zwischen den ersten und zweiten DC-Potentialtöpfen, um zu ermöglichen, dass sich positive Peptidionen, die im ersten DC-Potentialtopf begrenzt sind, und negativ geladene Reagenspartikel, die im zweiten DC-Potentialtopf begrenzt sind, vermischen; und
ein Massenspektrometriemodul zum Analysieren zumindest mancher Fragmente der positiven Peptidionen, die aus der Mischung extrahiert werden, wobei die zweiten Ionen aus dem Reagens abgeleitet werden.

## Revendications

1. Utilisation d'un composé en tant que réactif de dissociation pour transfert d'électrons pour spectrométrie de masse, dans laquelle le composé est un composé aromatique substitué ayant un facteur de Franck-Condon entre 0,1 et 1,0 et une affinité électronique ayant une valeur positive entre 0,1 et 200 kJ/mol, et dans laquelle soit le noyau aromatique est substitué par un ou plusieurs substituants consistant en fractions cyano, nitroso, carboxyl, iodo, aldéhyde et acétoxy, soit le composé est le 1,4-nitrotoluène.

2. Utilisation selon la revendication 1, dans laquelle ledit au moins un substituant est cyano.

3. Utilisation selon l'une des revendications 1 et 2, dans laquelle le réactif a une affinité électronique avec une valeur positive entre 0,1 à 150 kJ/mol, de préférence 0,1 à 100 kJ/mol.

4. Utilisation selon l'une quelconque des revendications précédentes, dans laquelle le noyau aromatique est choisi dans le groupe consistant en phényle, pyridinyle, pyrimidinyle, triazolyle, styrényle et picolinyle.

5. Utilisation selon l'une quelconque des revendications précédentes, dans laquelle le composé est choisi dans le groupe consistant en :
1,4-dicyanobenzène
1,3-dicyanobenzène
1,4-nitrotoluène
1,3-benzènedicarboxaldéhyde (isophtalaldéhyde)
1,2 ortho-nitrosotoluène
benzaldéhyde oxime
nitrosobenzène
3-pyridinecarbonitrile
éthyl cis (bêta cyano) acrylate
1,2,4,5-cyanobenzène
1,4-benzènedicarboxaldéhyde (téréphtalaldéhyde)
3-acétobenzonitrile
4-formyl benzonitrile

6. Utilisation selon l'une quelconque des revendications précédentes, dans laquelle le composé est choisi dans le groupe consistant en :
1,2-dicyanoéthylène
5-cyano-1,2,4-triazole (1,2,4-triazole-5-carbonitrile)
2,5-dicyanopyrimidine
3,5-dicyanopyridine
5-méthylpyridine 3-carbonitrile
5-pyrimidinecarbonitrile
2,4-dicyanopyrimidine
3,5-dicyanotoluène
4-cyanostyrène (4-éthynylbenzonitrile)
benzaldéhyde
2-cyanostyrène
1,3,4,5-cyanobenzène
3-méthyl picolinonitrile
4-vinylpyridine
4-pyrimidine carbonitrile
2-pyrimidine carbonitrile
4-acétobenzonitrile
2-pyrazinecarbonitrile
3-acétopyridine
4-cyanonitrobenzène
3-chlorobenzaldéhyde
3-cyanonitrobenzène
1,4-diacétylbenzène
1,2-dicyanobenzène
3-nitrosopyridine
4-acétopyridine
4-chloroacétophénone
3-chloroacétophénone
3,5-dichlorobenzonitrile
1,3-méta-nitrosotoluène
1,2,3-tricyanobenzène
3,5-chloroacétophénone
4-nitrosopyridine
3-amino-4-cyanopyridine
2-amino-4-cyanopyridine
2-acétopyridine
2-nitrosopyridine
4-amino-2-cyanopyridine
2-acétobenzonitrile
4-amino-3-cyanopyridine
1,3-cyclohexanedione
4-amino-5-pyrimidine carbonitrile
4,5-dicyanoimidazole
3-thiophène carbonitrile
2-cyanoéthyl acrylate
diaminomaléonitrile
2-cyanothioacétamide
acide phtalique
4-chloropyridine
2,4,6-CHT-3,6-carbonitrile
acétyl malonitrile
éthyl-2-cyanoacrylate
acide 2-cyano-3-méthyl but-2-énoique
1,2-dicyanoéthyne (acétylène dinitrile).

7. Utilisation selon l'une quelconque des revendications 1 à 6 dans laquelle le composé forme un ion radicalaire lors de l'utilisation.

8. Procédé de caractérisation d'un analyte comprenant les étapes consistant à se procurer un premier apport de cations d'analyte à l'iade d'ionisation par électronébulisation ;
se procurer un second apport d'anions de réactif à l'aide d'une source d'ionisation ;
combiner les premier et second apports ;
exécuter une technique de spectrométrie de masse (MS) sur les apports combinés ;
dans lequel le réactif est un composé aromatique substitué ayant un facteur de Franck-Condon entre 0,1 et 1,0 et une affinité électronique ayant une valeur positive comprise entre 0,1 et 200 kJ/mol, et dans lequel soit le noyau aromatique est substitué par un ou plusieurs substituants consistant en fractions cyano, nitroso, carboxyl, iodo, aldéhyde et acétoxy, soit le composé est 1,4-nitrotoluène.

9. Procédé selon la revendication 8, dans lequel l'analyte est une biomolécule.

10. Procédé selon la revendication 9, dans lequel l'analyte est un polypeptide.

11. Procédé selon l'une quelconque des revendications 8 à 10, comprenant l'étape d'utilisation d'un dispositif de dissociation par transfert d'électrons, adapté pour que : (a) dans un mode de fonctionnement, une ou plusieurs premières tensions ou potentiels en courant continu transitoires ou une ou plusieurs premières formes d'onde de tension ou potentiel en courant continu transitoires sont appliquées à au moins une partie d'une pluralité d'électrodes de façon à entraîner ou solliciter au moins une partie des ions de produit ou fragment le long d'au moins une partie de la longueur axiale des électrodes dans une direction différente d'une première direction ; et/ou (b) dans un mode de fonctionnement, le ou les secondes tensions ou potentiels en courant continu transitoires ou une ou plusieurs secondes formes d'onde de tension ou potentiel en courant continu transitoires sont appliquées à au moins une partie de la pluralité d'électrodes de façon à entraîner les ions de produit ou fragmentés le long et/ou à travers au moins une partie de la longueur axiale des électrodes dans une direction différente de la seconde direction.

12. Procédé selon l'une quelconque des revendications 8 à 11, comprenant les étapes consistant à :
se procurer un dispositif de dissociation par transfert d'électrons comprenant une pluralité d'électrodes empilées ;
appliquer une ou plusieurs premières tensions ou potentiels en courant continu transitoires ou une ou plusieurs premières formes d'onde de tension ou potentiel en courant continu transitoires à au moins une partie de la pluralité d'électrodes de façon à entraîner au moins une partie de premiers ions le long d'au moins une partie de la longueur axiale du guide d'ions dans une première direction ; et appliquer une ou plusieurs secondes tensions ou potentiels en courant continu transitoires ou une ou plusieurs secondes formes d'onde de tension ou potentiel en courant continu transitoires à au moins une partie de la pluralité d'électrodes de façon à entraîner au moins une partie de seconds ions le long d'au moins une partie de la longueur axiale du guide d'ions dans une seconde direction différente, les seconds ions étant issus dudit réactif.

13. Procédé selon l'une quelconque des revendications 8 à 12, comprenant l'étape consistant à se procurer :
un dispositif de fragmentation, comprenant une pluralité d'électrodes disposées en série le long d'un axe longitudinal du dispositif de fragmentation et définissant une région de manipulation d'ions ;
des moyens pour appliquer au moins une première tension en courant continu à au moins deux électrodes contiguës de la pluralité d'électrodes pour définir un premier puits de potentiel en courant continu, et pour appliquer au moins une seconde tension en courant continue à au moins deux électrodes contiguës différentes de la pluralité d'électrodes pour définir un second puits de potentiel en courant continu ;
des moyens pour réduire une barrière de potentiel en courant continu entre les premier et second puits de potentiel en courant continu pour permettre à des ions peptidiques positifs, confinés dans le premier puits de potentiel en courant continu, et à des particules de réactif chargées négativement, confinées dans un second puits de potentiel en courant continu, de se mélanger ; et
un module de spectrométrie de masse pour analyser au moins une partie des fragments des ions peptidiques positifs extraits du mélange, les seconds ions étant issus dudit réactif.
